# EUROPÄISCHE PATENTSCHRIFT

(11) **EP 0 948 950 B1**
(45) Veröffentlichungstag und Bekanntmachung des Hinweises auf die Patenterteilung: **08.03.2006**
(21) Anmeldenummer: 99106118.5
(22) Anmeldetag: 01.04.1999
(51) Int. Cl.: A61F 13/02, B32B 27/08, B32B 3/24

(54) **Trägermaterial für Pflaster und Wundverbände**
Carrier for plasters and wound dressings
Support pour emplâtres et pansements

(30) Priorität: 08.04.1998 DE 19815762
(43) Veröffentlichungstag der Anmeldung: 13.10.1999
(73) Patentinhaber: Beiersdorf Aktiengesellschaft, 20245 Hamburg (DE)
(72) Erfinder: Trotter, Sebastian, 21244 Buchholz (DE)

(56) Entgegenhaltungen:
- EP-A- 0 446 431
- EP-A- 0 651 984

## Beschreibung

Die Erfindung betrifft ein flexibles mehrschichtiges Laminat zur Verwendung als Trägermaterial in Pflastern und Wundabdeckungen, deren Applikation ohne Hilfsträger erfolgen kann.

Flexible Trägermaterialien werden bevorzugt eingesetzt, wenn Pflaster nach dem Applizieren den Körperbewegungen folgen und dabei keinen zu großen Widerstand entgegenbringen soll.

Aus der DE 43 14 834 C2 ist ein Verbandmaterial auf Folienbasis bekannt, das auf der einen Seite mit einem Trägermaterial abgedeckt ist, das die gleiche Größe wie die Folie besitzt und mindestens eine Griffleiste aufweist, und auf der anderen Seite mit einer selbstklebenden Schicht versehen ist. Die Griffleisten sind dabei innerhalb seiner Umfangsbegrenzung angeordnet. Die Folie besteht bevorzugt aus Polyurethan, das Trägermaterial aus Polyethylen.
Bei der Applizierung des Verbandmaterials wird mittels der Griffleisten das Trägermaterial von der Folie, die durch die selbstklebende Schicht auf der Haut haften bleibt, entfernt.
Insbesondere bei Anwendungen im Bereich der Gelenke sind die Vorteile von Produkten, wie sie in der DE 43 14 834 C2 zur Verfügung gestellt werden, hinsichtlich der Flexibilität bekannt.
Weiterhin wird durch die Verwendung von PU-Trägerfolien eine hohe Wasserdampfdurchlässigkeit erzielt.
Das Produktdesign weist jedoch eine Vielzahl von Nachteilen auf. Durch die vorgesehenen Griffleisten zum Entfernen des Hilfsträgers entstehen zusätzlicher Abfall und hohe Kosten, dann wird die fachgerechte Applikation, d.h. das notwendige Entfernen des Hilfsträgers, vom Kunden nicht immer ad hoc verstanden. Des weiteren steht der unflexible Hilfsträger einem faltenfreien und damit wasser- beziehungsweise keimdichten Applizieren zum Beispiel am Finger häufig im Wege. Schließlich stört der Hilfsträger gerade beim Applizieren am Finger im Falle einer Überlappung.
Ein vergleichbares Verbandmaterial ist aus der DE 40 26 755 A bekannt. Hier weist das Trägermaterial allerdings zwei Griffleisten auf, die über das Verbandmaterial hinausstehen.

In der DE 40 07 891 A wird ein Trägermaterial für medizinische Zwecke offenbart, das aus einem Laminat besteht, das seinerseits aus einer ersten polymeren Filmschicht, einer zweiten, auf der ersten erzeugten polymeren Filmschicht und einer dritten, in der zweiten Schicht zumindest teilweise eingebetteten Schicht aus einem makropörosen textilen Material zusammengesetzt ist.

In der EP 0 673 657 A1 ist ein weiteres Laminat beschreiben, das sich aus drei Schichten zusammensetzt. Auf ein Vlies wird einseitig eine selbstklebende Beschichtung aufgebracht. Auf die gegenüberliegende Vliesseite wird schließlich ein wasserdampfdurchlässiger und weitgehend wasserundurchlässiger Film aufgetragen.

Aufgabe der Erfindung war es, die Nachteile des Standes der Technik zu vermeiden, zumindest zu vermindern. Insbesondere ist es die Aufgabe der Erfindung, ein Trägermaterial für medizinische Zwecke zur Verfügung zu stellen, das sehr flexibel und wasserdampfdurchlässig ist und dennoch eine hohe Festigkeit aufweist.

Gelöst wird diese Aufgabe durch ein Trägermaterial für medizinische Zwecke bestehend aus einem Laminat, wie dies näher im Hauptanspruch gekennzeichnet ist. Gegenstand der Unteransprüche sind besonders vorteilhafte Ausführungsformen des Laminats. Des weiteren betrifft die Erfindung ein Verfahren zur Herstellung des Laminats.

Demgemäß betrifft die Erfindung ein Trägermaterial für medizinische Zwecke bestehend aus einem Laminat aus einer ersten polymeren Filmschicht, einer flexiblen Netzfolie und einer zumindest partiell aufgetragenen selbstklebenden Beschichtung, wobei die Netzfolie in der selbstklebenden Beschichtung teilweise eingebettet und auf diese Weise darin verankert ist.

Zwischen der ersten polymeren Schicht und der selbstklebenden Beschichtung ist vorzugsweise eine zweite polymere Schicht vorhanden.

Als vorteilhaft hat sich herausgestellt, wenn die erste und die zweite polymere Schicht aus einem elastischen aliphatischen Polyesterpolyurethan bestehen.
Weiterhin ist vorteilhaft, wenn die erste und zweite polymere Schicht eine Dicke von jeweils 0,005 mm bis 0,035 mm aufweisen.

Die Netzfolie besteht vorzugsweise aus Polyethylen, Polypropylen, Polyamid, Polyurethan, EVA, Polystyrol (auch High Impact Polystyrol (HIPS)) oder Kompositionen aus den genannten Polymeren. Darüber hinaus kann die Netzfolie auch eingefärbt sein, um besondere optische Effekte des Laminats zu erzielen.

Vorzugsweise weist die Netzfolie folgende Charakteristika auf:
- eine Dicke von 0,01 mm bis 0,07 mm
- ein Flächengewicht von 5 g/m² bis 40 g/m²
- eine offene Fläche von 30% bis 70% und/oder
- eine Lochgröße von 0,3 mm bis 1,5 mm

Um die geforderte Stabilität des Laminats zu gewährleisten, sollte die selbstklebende Beschichtung mit einem Flächengewicht von 20 g/m² bis 100 g/m² aufgetragen werden.

Besonders geeignet ist das erfindungsgemäße Trägermaterial zur Herstellung von Pflastern, welche gegebenenfalls auf der selbstklebenden Beschichtung eine Wundauflage aufweisen.

Schließlich umfaßt die Erfindung auch ein Verfahren zur Herstellung des Trägermaterials.
Danach wird zunächst die erste polymere Schicht auf einem Hilfsträger erzeugt. Gegebenenfalls wird die zweite polymere Schicht auf der ersten Schicht erzeugt.
Parallel wird dazu auf einen klebstoffabweisend ausgerüsteten Zwischenträger die Klebemasse zumindest partiell ausgestrichen, und zwar wird die Klebemasse als Lösung, Dispersion oder Schmelze auf den klebstoffabweisend ausgerüsteten Zwischenträger, zum Beispiel ein silikonisiertes Kraftpapier oder eine silikonisierte Folie, ausgestrichen, dann in die noch flüssige Klebemasse die Netzfolie einkaschiert beziehungsweise eingelegt.

Dabei sinkt die Netzfolie mehr oder weniger tief in die noch Lösungsmittel enthaltene Masse beziehungsweise noch flüssige Schmelze ein. Im Trockenkanal verdampft das Lösungsmittel und die Klebemasse umschließt die Netzfolie, so daß sich der Verbund verfestigt. Im Falle eines Schmelzhaftklebstoffes, verfestigt sich der Verbund durch Abkühlung der Klebemasse.
Daran schließt sich das Zusammenkaschieren der ersten und gegebenenfalls vorhandenen zweiten polymeren Schicht mit dem Zwischenlaminat aus Netzfolie, Klebebeschichtung und Zwischenträger an, wobei das daraus resultierende Laminat noch verfestigt wird, insbesondere kalandert.
Falls erforderlich wird die Masse mittels UV-Strahlung etc. vemetzt.
Der zur Herstellung der ersten und zweiten polymeren Schicht verwendete Zwischenträger wird dekaschiert und die Materialbahn kann auf sich selbst gewickelt und nachfolgend zu Mutterrollen der gewünschten Breite aufgeschnitten werden.
Anschließend erfolgt die Konfektionierung zu gebrauchsfertigen Einzelprodukten mit oder ohne Wundauflage, wobei das Endprodukt gegebenenfalls γ-sterilisiert wird.

Durch das erfindungsgemäße Trägermaterial wird ein Laminat zu Verfügung gestellt, in dem die flexible PU-Folie durch eine in die Klebemasse eingearbeitete flexible Netzfolie soweit unterstützt wird, daß eine Applikation ohne Hilfsträgerfolie möglich ist und die geforderten Produkteigenschaften beibehalten werden.

Das Laminat weist viele Vorteile auf:
1. Es ist kein Hilfsträger und damit auch keine Griffleiste mehr erforderlich, was gegenüber den aus dem Stand der Technik bekannten Produkte zur einer Reduzierung der Kosten führt.
2. Der Verwender erhält ein Produkt, das ohne weitere Erklärungen appliziert werden kann.
3. Die einfache Handhabung erlaubt wie ein herkömmliches Produkt auch Überlappungen beim Verkleben, zum Beispiel bei Anwendung am Finger.
4. Das Produkt erscheint weniger umweltbelastend, da der Abfall reduziert wird.
5. Durch die neue Optik (das Netz kann eingefärbt werden) ist die Innovation sofort erkennbar und vermittelbar.

Im folgenden soll die Erfindung anhand eines Beispiels näher erläutert werden, ohne damit die Erfindung unnötig einschränken zu wollen.

### Beispiel 1

### 1a) Herstellung des Zwischenproduktes

Auf eine mattierte PE-Folie (zum Beispiel LDPE Waloplast matt 230 F 80 der Fa. Wolff Walsrode) wird eine anionische aliphatische Polyesterpolyurethan Dispersion (Impranil®-Typen der Fa. Bayer AG) in einer Dicke von 0,015 bis 0,02 mm ausgestrichen und in einem stufenweise beheizten Kanal bei Temperaturen von 20 °C bis 90 °C blasenfrei getrocknet, so daß sich eine Filmschicht bildet. Anschließend wird in gleicher Weise und Dicke eine weitere Filmschicht auf die bereits bestehende aufgebracht.

### 1b) Herstellung Verbund Netzfolie/Selbstklebemasse

Auf eine silikonisierte Trennfolie (zum Beispiel Trennfolie farblos LF D 70 der Fa. Laufenberg) wird eine UV-vernetzbare Selbstklebemasse gemäß der DE P 27 43 979 ausgestrichen, so daß nach dem Trocknen eine Schichtstärke von 50 g/m² erzielt wird. Unmittelbar hinter der Beschichtungsvorrichtung wird eine Netzfolie (zum Beispiel Netz RX 27 der Fa. Smith&Nephew) in die flüssige Selbstklebemasse gelegt, so daß diese ganz oder teilweise in der Lösung versinkt.

Zum Abdunsten des Lösungsmittels wird die gesamte Bahn in einem stufenweise beheizten Trockenkanal bei Temperaturen von 20 °C bis 80 °C getrocknet. Nach Verlassen des Trockenkanals wird das unter 1a) beschriebene Zwischenprodukt mit der PU-Folienseite auf die offene Seite der Selbstklebemasse kaschiert.

Das Verbundprodukt wird mittels UV bestrahlt, um die Selbstklebemasse zu vemetzen. Anschließend wird die PE-Hilfsfolie ausgedeckt und das Laminat zu Ballen aufgewickelt. Die Ballen werden zu Rollen in unterschiedlicher Breite aufgeschnitten, die noch vorhandene Trennfolie wird entfernt, eine Wundauflage aufgelegt und erneut mit Trennpapier eingedeckt. Aus der so erhaltenen Bahn werden Einzelpflaster der gewünschten Abmessungen gestanzt und einzeln eingesiegelt.

## Patentansprüche

1. Trägermaterial für medizinische Zwecke bestehend aus einem Laminat aus einer ersten polymeren Filmschicht, einer flexiblen Netzfolie und einer zumindest partiell aufgetragenen selbstklebenden Beschichtung, wobei die Netzfolie in der selbstklebenden Beschichtung teilweise eingebettet und auf diese Weise darin verankert ist, **dadurch gekennzeichnet, dass** die selbstklebende Beschichtung mit einem Flächengewicht von 20 g/m² bis 100 g/m² aufgetragen ist.

2. Trägermaterial nach Anspruch 1, **dadurch gekennzeichnet, daß** zwischen der ersten polymeren Schicht und der selbstklebenden Beschichtung eine zweite polymere Schicht vorhanden ist.

3. Trägermaterial nach Anspruch 1, **dadurch gekennzeichnet, daß** die erste und die zweite polymere Schicht aus einem elastischen aliphatischen Polyesterpolyurethan bestehen.

4. Trägermaterial nach Anspruch 1, **dadurch gekennzeichnet, daß** die erste und zweite polymere Schicht eine Dicke von 0,005 mm bis 0,035 mm aufweisen.

5. Trägermaterial nach Anspruch 1, **dadurch gekennzeichnet, daß** die Netzfolie aus Polyethylen, Polypropylen, Polyamid, Polyurethan, EVA, Polystyrol oder aus Kompositionen der genannten Polymeren besteht.

6. Trägermaterial nach Anspruch 1, **dadurch gekennzeichnet, daß** die Netzfolie
eine Dicke von 0,01 mm bis 0,07 mm
ein Flächengewicht von 5 g/m² bis 40 g/m²,
eine offene Fläche von 30% bis 70% und/oder
eine Lochgröße von 0,3 mm bis 1,5 mm aufweist.

7. Trägermaterial nach Anspruch 1, **dadurch gekennzeichnet, daß** aus dem Trägermaterial Pflaster gestanzt werden, welche gegebenenfalls auf der selbstklebenden Beschichtung eine Wundauflage aufweisen.

8. Verfahren zur Herstellung eines Trägermaterials nach mindestens einem der vorgehenden Ansprüche, indem
die erste polymere Schicht auf einem Hilfsträger erzeugt wird,
gegebenenfalls die zweite polymere Schicht danach auf der ersten Schicht erzeugt wird, auf einen klebstoffabweisend ausgerüsteten Zwischenträger die Klebemasse zumindest partiell ausgestrichen wird,
in die noch flüssige Klebemasse die Netzfolie einkaschiert wird,
die erste beziehungsweise zweite polymere Schicht mit dem Zwischenlaminat aus Netzfolie, Klebebeschichtung und Zwischenträger zusammenkaschiert und das entstehende Laminat verfestigt wird.

## Claims

1. Backing material for medical purposes, consisting of a laminate comprising a first polymeric film layer, a flexible net film and an at least partially applied self-adhesive coating, the net film being partly embedded in the self-adhesive coating and so anchored therein, **characterized in that** the self-adhesive coating is applied with a weight per unit area of from 20 to 100 g/m².

2. Backing material according to Claim 1, **characterized in that** there is a second polymeric layer between the first polymeric layer and the self-adhesive coating.

3. Backing material according to Claim 1, **characterized in that** the first and the second polymeric layers consist of an elastic aliphatic polyesterpolyurethane.

4. Backing material according to Claim 1, **characterized in that** the first and second polymeric layers have a thickness of from 0.005 to 0.035 mm.

5. Backing material according to Claim 1, **characterized in that** the net film consists of polyethylene, polypropylene, polyamide, polyurethane, EVA, polystyrene, or compositions of the said polymers.

6. Backing material according to Claim 1, **characterized in that** the net film has
a thickness from 0.01 to 0.07 mm
a basis weight from 5 to 40 g/m²
an open area of 30 to 70%, and/or
a mesh size from 0.3 to 1.5 mm.

7. Backing material according to Claim 1, **characterized in that** plasters are punched from the backing material and these plasters may, if desired, have a wound contact material on the self-adhesive coating.

8. Process for producing a backing material according to at least one of the preceding claims, in which
the first polymeric layer is produced on an auxiliary support,
if desired, the second polymeric layer is then produced on the first layer,
the adhesive composition is applied at least partly to an intermediate support which has been given an anti-adhesive treatment,
the net film is laminated into the still liquid adhesive composition,
the first or second polymeric layer, respectively, is laminated together with the intermediate laminate comprising net film, adhesive coating and intermediate support, and
the resultant laminate is consolidated.

## Revendications

1. Matériau support destiné à des fins médicales, constitué par un laminé constitué par une première couche de feuille polymère, une feuille à mailles souple et un revêtement auto-adhésif appliqué au moins partiellement, la feuille à mailles étant noyée partiellement dans le revêtement auto-adhésif et ancrée de cette manière dans celui-ci, **caractérisé en ce que** le revêtement auto-adhésif est appliqué à raison d'un poids surfacique de 20 g/m² à 100 g/m².

2. Matériau support selon la revendication 1, **caractérisé en ce qu'**il existe entre la première couche polymère et le revêtement auto-adhésif une deuxième couche polymère.

3. Matériau support selon la revendication 1, **caractérisé en ce que** la première et la deuxième couche polymère sont constituées par un polyester-polyuréthane aliphatique élastique.

4. Matériau support selon la revendication 1, **caractérisé en ce que** la première et la deuxième couche polymère présentent une épaisseur de 0,005 mm à 0,035 mm.

5. Matériau support selon la revendication 1, **caractérisé en ce que** la feuille à mailles est constituée par du polyéthylène, du polypropylène, du polyamide, du polyuréthane, du EVA, du polystyrène ou par des compositions des polymères mentionnés.

6. Matériau support selon la revendication 1, **caractérisé en ce que** la feuille à mailles présente une épaisseur de 0,01 mm à 0,07 mm, un poids surfacique de 5 g/m² à 40 g/m², une surface ouverte de 30% à 70% et une taille des trous de 0,3 mm à 1,5 mm.

7. Matériau support selon la revendication 1, **caractérisé en ce qu'**on découpe des emplâtres du matériau support, qui présentent le cas échéant un pansement sur le revêtement auto-adhésif.

8. Procédé pour la réalisation d'un matériau support selon au moins l'une quelconque des revendications précédentes, dans lequel
on produit la première couche polymère sur un support auxiliaire,
on produit ensuite le cas échéant la deuxième couche polymère sur la première couche, on étale au moins partiellement la masse adhésive sur un support intermédiaire apprêté de manière anti-adhésive,
on noie la feuille à mailles dans la masse adhésive encore liquide,
on assemble par collage la première resp. la deuxième couche polymère avec le laminé intermédiaire constitué par la feuille à mailles, le revêtement adhésif et le support intermédiaire et
on solidifie le laminé formé.
